# EUROPÄISCHE PATENTSCHRIFT

(11) **EP 1 483 226 B1**
(45) Veröffentlichungstag und Bekanntmachung des Hinweises auf die Patenterteilung: **07.09.2005**
(21) Anmeldenummer: 03724908.3
(22) Anmeldetag: 28.02.2003
(51) Int. Cl.: C07C 43/174, C07C 41/16

(54) **VERBESSERTES VERFAHREN ZUR HERSTELLUNG VON 4-(6-BROMHEXYLOXY)-BUTYLBENZOL**
IMPROVED METHOD FOR THE PRODUCTION OF 4-(6-BROMOHEXYLOXY) BUTYLBENZENE
PROCEDE AMELIORE DE PRODUCTION DE 4-(6-BROMOHEXYLOXY)-BUTYLBENZENE

(30) Priorität: 05.03.2002 DE 10209583
(43) Veröffentlichungstag der Anmeldung: 08.12.2004
(73) Patentinhaber: Boehringer Ingelheim Pharma GmbH & Co.KG, 55216 Ingelheim am Rhein (DE)
(72) Erfinder: HEITGER, Helmut, 55218 INGELHEIM (DE); MEYER, Oliver, 55452 DORSHEIM (DE)
(86) Internationale Anmeldenummer: PCT/EP2003/002054
(87) Internationale Veröffentlichungsnummer: WO 2003/074458

(56) Entgegenhaltungen:
- EP-A- 1 132 373
- WO-A-95/19336
- DE-A- 3 414 752
- Y. RONG: "A new synthetic approach to salmeterol" SYNTHETIC COMMUNICATIONS., Bd. 29, Nr. 12, 1999, Seiten 2155-2162, XP001018659 MARCEL DEKKER, INC., BASEL., CH ISSN: 0039-7911
- R. V. BONNERT: "Dual D2-receptor and beta2-adrenoceptor agonists for the treatment of airway diseases. 1. Discovery and biological evaluation of some 7-(2-aminoethyl)-4-hydroxybenzothiazol-2(3 H)-one analogues" JOURNAL OF MEDICINAL CHEMISTRY, Bd. 41, Nr. 25, 1998, Seiten 4915-4917, XP002928403 WASHINGTON US

## Beschreibung

Die vorliegende Erfindung betrifft ein verbessertes Verfahren zur Herstellung von 4-(6-Bromhexyloxy)-butylbenzol durch Umsetzung von 4-Phenylbutanol mit 1,6-Dibromhexan in Gegenwart einer Base und eines Phasentransferkatalysators, sowie die Verwendung des so hergestellten 4-(6-Bromhexyloxy)-butylbenzol zur Herstellung von Salmeterol in an sich bekannter Weise.

### Hintergrund der Erfindung

4-(6-Bromhexyloxy)-butylbenzol ist ein wertvolles Zwischenprodukt zur Herstellung des Wirkstoffes Salmeterol, welcher als Bronchodilatator zur Behandlung von Asthma oder chronischer Bronchitis eingesetzt wird.

Nach der Lehre der deutschen Patentanmeldung DE 34 14 752 wird 4-(6-Bromhexyloxy)-butylbenzol durch Umsetzung von 4-Phenylbutanol mit 1,6-Dibromhexan mit einer Natriumhydrid Dispersion als Base hergestellt. Aus Sicherheitsgründen ist eine Produktion im technischen Maßstab unter Einsatz von Natriumhydrid Dispersionen kaum durchführbar.

Die europäische Patentanmeldung EP 1 132 373 beschreibt ein Laborverfahren zur Herstellung von 4-(6-Bromhexyloxy)-butylbenzol, bei dem eine Mischung bestehend aus 4-Phenylbutanol, 1,6-Dibromhexan, Kaliumhydroxid und Tetrabutylammonium Hydrogensulfat bei Raumtemperatur 20 Stunden gerührt wird. Dieses Laborverfahren lässt sich allerdings nicht im technischen Maßstab durchführen, da diese Reaktion stark exotherm ist, was zu einem starken Ansteigen der Reaktionstemperatur führt, wobei schwer rührbare Rückstände und' die Ausbeute mindernde Nebenprodukte (Eliminierungsprodukte) entstehen.

Der vorliegenden Erfindung liegt somit die Aufgabe zu Grunde, ein Verfahren aufzuzeigen, welches es ermöglicht 4-(6-Bromhexyloxy)-butylbenzol in guten Ausbeuten im großtechnischen Maßstab herzustellen und dabei die bei aus dem Stand der Technik bekannten Verfahren auftretenden Nachteile zu vermeiden.

### Detaillierte Beschreibung der Erfindung

Überraschenderweise wurde nun gefunden, dass 4-(6-Bromhexyloxy)-butylbenzol in guten Ausbeuten und im technischen Maßstab durch Umsetzung von 4-Phenylbutanol mit 1,6-Dibromhexan in Gegenwart einer Base und eines Phasentransferkatalysators hergestellt werden kann, wenn man 4-Phenylbutanol in einem Verdünnungsmittel zu einem Gemisch bestehend aus 1,6-Dibromhexan, einer Base, einem Phasentransferkatalysator und einem Verdünnungsmittel hinzufügt.

Gegenstand der Erfindung ist somit ein verbessertes Verfahren zur Herstellung von 4-(6-Bromhexyloxy)-butylbenzol durch Umsetzung von 4-Phenylbutanol mit 1,6-Dibromhexan in Gegenwart einer Base und eines Phasentransferkatalysators, wobei man 4-Phenylbutanol in einem Verdünnungsmittel zu einem Gemisch bestehend aus 1,6-Dibromhexan, einer Base, einem Phasentransferkatalysator und einem Verdünnungsmittel dosiert.

Ein weiterer Gegenstand der Erfindung ist ein Verfahren zur Herstellung von Salmeterol wobei 4-(6-Bromhexyloxy)-butylbenzol wie oben hergestellt und in an sich bekannter Weise weiter verarbeitet wird.

Bevorzugte Ausführungsformen des erfindungsgemäßen Verfahren sind Verfahren, wobei:
(A) man als Base ein Alkalimetallhydroxid, wie zum Beispiel Lithiumhydroxid, Natriumhydroxid oder Kaliumhydroxid, insbesondere gepulvertes Kaliumhydroxid einsetzt;
(B) man als Phasentransferkatalysator (PTK) ein Tetraalkylammonium- oder Tetraalkylphosphoniumsalz, wobei die jeweiligen Alkylgruppen gleich oder verschieden sein können, wie zum Beispiel Salze von Tetraoktylammonium, Methyltrioktylammonium, Tetramethylammonium, Tetraethylammonium, Tetrahexylammonium Aliquat 175 (Tributylmethylammonium) oder Aliquat 336 (Methyltrioktylammonium) verwendet. Vorzugsweise ist der PTK ein Tetraalkylammonium Halogenid, ein Tetraalkylammonium Sulfat, ein Tetraalkylammonium Hydrogensulfat, ein Tetraalkylammonium Nitrat oder ein Tetraalkylammonium Phosphat, insbesondere ein Tetraalkylammonium Hydrogensulfat, ganz besonders bevorzugt Tetra-*n*-butylammonium Hydrogensulfat.
   Der Begriff "Alkyl" wie er vor- und nachstehend im Bezug auf den Phasentransferkatalysator verwendet wird umfasst geradkettige und verzweigte Alkylgruppen mit 1 bis 8, vorzugsweise 2 bis 6, insbesondere 4 Kohlenstoffatomen. Als bevorzugte Alkylgruppen seien somit die Ethyl-, *n*-Propyl-, *i*-Propyl-, *n*-Butyl-, 2-Butyl, *tert*-Butyl-, *n*-Pentyl-, 2-Pentyl-, *neo*-Pentyl-, *n*-Hexyl- und 2-Hexylgruppe genannt. Ganz besonders bevorzugt ist die *n*-Butylgruppe.
   Weitere bevorzugte Ausführungsformen des erfindungsgemäßen Verfahren sind Verfahren, wobei:
(C) man als Verdünnungsmittel einen aromatischen Kohlenwasserstoff, vorzugsweise Benzol, Toluol oder Xylol, insbesondere Toluol, oder einen gegebenenfalls halogenierten aliphatischen Kohlenwasserstoff, vorzugsweise Dichlormethan, Chloroform, Tetrachlorkohlenstoff oder Dichlorethan, insbesondere Dichlormethan verwendet;
(D) man die Umsetzung in einem Temperaturbereich von -10 °C bis + 30 °C, vorzugsweise von 0 ° C bis 28 °C, insbesondere von 20 bis 25 °C durchführt;
(E) man das 4-Phenylbutanol innerhalb von 10 bis 240 Minuten, vorzugsweise 15 bis 60 Minuten zudosiert und die erhaltene Reaktionsmischung noch weitere 30 bis 180, vorzugsweise 45 bis 150 Minuten nachrührt;
(F) man 0,75 bis 2,5 Äquivalente, vorzugsweise 1,5 bis 2,2 Äquivalente, insbesondere etwa 2 Äquivalente 1,6-Dibromhexan bezogen auf 1 Äquivalent 4-Phenylbutanol einsetzt;
(G) man 2,5 bis 5,5 Äquivalente, vorzugsweise 2,8 bis 5,2 Äquivalente, insbesondere 3,0 bis 4,0 Äquivalente an Base bezogen auf 1 Äquivalent 4-Phenylbutanol einsetzt;
(H)man 0,01 bis 0,5 Äquivalente, vorzugsweise 0,02 bis 0,2 Äquivalente, insbesondere 0,05 bis 0,15 Äquivalente an Phasentransferkatalysator bezogen auf 1 Äquivalent 4-Phenylbutanol einsetzt;
(I) das Gemisch aus 1,6-Dibromhexan, einer Base, einem Phasentransferkatalysator und einem Verdünnungsmittels 1,5 bis 4,0 Volumenteile, vorzugsweise 2,0 bis 3,0 Volumenteile, insbesondere 2,2 bis 2,8 Volumenteile an Verdünnungsmittel bezogen auf ein Volumenteil 1,6-Dibromhexan enthält;
(J) das Gemisch aus 4-Phenylbutanol und Verdünnungsmittel 1,5 bis 10 Volumenteile, vorzugsweise 2,0 bis 6,0 Volumenteile, insbesondere 3,0 bis 5,0 Volumenteile an Verdünnungsmittel bezogen auf ein Volumenteil 4-Phenylbutanol enthält;
(K) man nach Beendigung der Umsetzung Wasser zur Reaktionsmischung hinzufügt, die Phasen trennt, die organische Phase mit Wasser wäscht, unter reduziertem Druck einengt und den Rückstand unter Hochvakuum fraktioniert destilliert.

In einer besonders bevorzugten Ausführungsform wird 1 Äquivalent 4-Phenylbutanol in dem etwa doppelten Volumen an Toluol innerhalb von 10 bis 60 Minuten zu einem Gemisch aus etwa 2 Äquivalenten 1,6-Dibromhexan, 3 bis 5 Äquivalenten KOH, etwa 0,1 Äquivalenten Tetra-*n*-butylammonium Hydrogensulfat und dem 4 bis 6 fachen Volumen an Toluol bezogen auf das Volumen von 4-Phenylbutanol bei einer Temperatur zwischen 20 und 30 °C unter Rühren zudosiert. Nach Beendigung der Zugabe wird mit Toluol nachgespült und 90 bis 360, vorzugsweise etwa 180 bis 240 Minuten bei einer Temperatur zwischen 20 und 30 °C nachgerührt. Anschließend wird Wasser hinzugefügt, die organische Phase abgetrennt und mit Wasser gewaschen. Die organische Phase wird eingeengt und der Rückstand im Hochvakuum fraktioniert destilliert.

Weitere vorteilhafte Aspekte der erfindungsgemäßen Vorgehensweise sind die hohe Raum-Zeit-Ausbeute beim vorliegenden Prozess sowie die hohe Ausbeute und Reinheit an 4-(6-Bromhexyloxy)-butylbenzol, welches direkt oder nach Destillation zu Salmeterol weiterverarbeitet werden kann.

Ausgehend von dem nach dem erfindungsgemäßen Verfahren hergestellten 4-(6-Bromhexyloxy)-butylbenzol erfolgt die Herstellung von Salmeterol in an sich bekannter Weise, wie zum Beispiel beschrieben in der DE 34 14 752 oder der EP 1 132 373.

Die nachfolgenden Beispiele dienen der Illustration exemplarisch durchgeführter Verfahren zur Herstellung von 4-(6-Bromhexyloxy)-butylbenzol. Sie sind lediglich als mögliche, exemplarisch dargestellte Vorgehensweisen zu verstehen, ohne die Erfindung auf deren Inhalt zu beschränken.

### Beispiel 1 4-(6-Bromhexyloxy)-butylbenzol

Zu einem Gemisch aus 509,6 ml (3,3 Mol) 1,6-Dibromhexan, 402 g gepulvertes (6,1 Mol KOH) Ätzkali, 5,6 g (0,165 Mol) Tetra-*n*-butylammonium Hydrogensulfat und 1250 ml Toluol wird bei einer Temperatur zwischen 20 und 25 °C unter Rühren ein Gemisch aus 247,5 ml (1,65 Mol) 4-Phenylbutanol und 500 ml Toluol innerhalb von 45 Minuten zudosiert.

Nach Beendigung der Zugabe wird mit 125 ml Toluol nachgespült und 2,5 Stunden bei einer Temperatur zwischen 20 und 25 °C nachgerührt. Anschließend werden 1875 ml Wasser hinzugefügt, die organische Phase wird abgetrennt und zweimal mit jeweils 1250 ml Wasser gewaschen. Die organische Phase wird am Rotationsverdampfer unter reduziertem Druck eingeengt und der Rückstand im Hochvakuum (p < 0,1 mbar) fraktioniert destilliert. Man erhält 408,5 g (79 % d. Th.) der Titelverbindung als farbloses Öl.

### Beispiel 2 4-(6-Bromhexyloxy)-butylbenzol

Zu einem Gemisch aus 20,2 ml (0,131 Mol) 1,6-Dibromhexan, 16 g gepulvertes (0,242 Mol KOH) Ätzkali, 2,2 g (0,0066 Mol) Tetra-*n*-butylammonium Hydrogensulfat und 50 ml Toluol wird bei einer Temperatur zwischen 25 und 30 °C unter Rühren ein Gemisch aus 10 ml (0,0655 Mol) 4-Phenylbutanol und 20 ml Toluol innerhalb von 15 Minuten zudosiert.

Nach Beendigung der Zugabe wird mit 5 ml Toluol nachgespült und 2 Stunden bei einer Temperatur zwischen 25 und 30 °C nachgerührt. Anschließend werden 75 ml Wasser hinzugefügt, die organische Phase wird abgetrennt und zweimal mit jeweils 50 ml Wasser gewaschen. Die organische Phase wird am Rotationsverdampfer unter reduziertem Druck eingeengt und der Rückstand im Hochvakuum (p < 0,1 mbar) fraktioniert destilliert. Man erhält 14,62 g (71 % d. Th.) der Titelverbindung als farbloses Öl.

### Vergleichsbeispiel 1 4-(6-Bromhexyloxy)-butylbenzol (entsprechend EP 1 132 373)

Zu einem Gemisch aus 5 ml (0,0323 Mol) 4-Phenylbutanol und 10,1 ml (0,0657 Mol) 1,6-Dibromhexan, werden unter Rühren 8 g gepulvertes (0,121 Mol KOH) Ätzkali hinzugefügt, wobei die Temperatur auf etwa 30 °C ansteigt. Anschließend werden 1,1 g (0,0033 Mol) Tetra-*n*-butylammonium Hydrogensulfat hinzugefügt, wobei die Temperatur auf etwa 65 °C ansteigt.

Nach Beendigung der Zugabe wird 20 Stunden bei Raumtemperatur nachgerührt. Die Reaktionsmischung wird filtriert, das Filtrat in 50 ml Diethylether aufgenommen und mit 50 ml Wasser versetzt. Die organische Phase wird abgetrennt und getrocknet. Die organische Phase wird am Rotationsverdampfer unter reduziertem Druck eingeengt und der Rückstand im Hochvakuum (p < 0,1 mbar) fraktioniert destilliert. Man erhält 5,8 g (56 % d. Th.) der Titelverbindung als farbloses Öl.

### Vergleichsbeisniel 2 4-(6-Bromhexyloxy)-butylbenzol

Zu einem Gemisch aus 20,2 ml (0,131 Mol) 1,6-Dibromhexan, 16 g gepulvertes (0,242 Mol KOH) Ätzkali und 2,2 g (0,0066 Mol) Tetra-*n*-butylammonium Hydrogensulfat werden bei einer Temperatur zwischen 20 und 30 °C unter Rühren 10 ml (0,0655 Mol) 4-Phenylbutanol innerhalb von 15 Minuten zudosiert. Die Reaktion war spontan exotherm und die Temperatur war kaum zu kontrollieren. Gegen Ende der Zugabe bildeten sich harte Klumpen und das Reaktionsgemisch ließ sich nicht mehr durchrühren.

## Patentansprüche

1. Verfahren zur Herstellung von 4-(6-Bromhexyloxy)-butylbenzol durch Umsetzung von 4-Phenylbutanol mit 1,6-Dibromhexan in Gegenwart einer Base und eines Phasentransferkatalysators, **dadurch gekennzeichnet, dass** man 4-Phenylbutanol in einem Verdünnungsmittel zu einem Gemisch bestehend aus 1,6-Dibromhexan, einer Base, einem Phasentransferkatalysator und einem Verdünnungsmittel dosiert.

2. Verfahren nach Anspruch 1, **dadurch gekennzeichnet, dass** man als Base gepulvertes Kaliumhydroxid einsetzt.

3. Verfahren nach Anspruch 1 oder 2, **dadurch gekennzeichnet, dass** man als Phasentransferkatalysator ein Tetraalkylammonium- oder Tetraalkylphosphoniumsalz verwendet.

4. Verfahren nach Anspruch 3, **dadurch gekennzeichnet, dass** man als Phasentransferkatalysator ein Tetraalkylammonium Hydrogensulfat verwendet.

5. Verfahren nach einem der Ansprüche 1 bis 4, **dadurch gekennzeichnet, dass** man als Verdünnungsmittel einen aromatischen Kohlenwasserstoff oder einen gegebenenfalls halogenierten aliphatischen Kohlenwasserstoff verwendet.

6. Verfahren nach Anspruch 5, **dadurch gekennzeichnet, dass** man als Verdünnungsmittel Toluol oder Dichlormethan verwendet.

7. Verfahren nach einem der Ansprüche 1 bis 6, **dadurch gekennzeichnet, dass** man die Umsetzung in einem Temperaturbereich von -10 °C bis + 30 °C durchführt.

8. Verfahren nach einem der Ansprüche 1 bis 7, **dadurch gekennzeichnet, dass** man das 4-Phenylbutanol innerhalb von 10 bis 240 Minuten zudosiert und die erhaltene Reaktionsmischung noch weitere 30 bis 180 Minuten nachrührt.

9. Verfahren nach einem der Anspruch 1 bis 8, **dadurch gekennzeichnet, dass** man 0,75 bis 2,5 Äquivalente 1,6-Dibromhexan bezogen auf 1 Äquivalent 4-Phenylbutanol einsetzt.

10. Verfahren nach einem der Ansprüche 1 bis 9, **dadurch gekennzeichnet, dass** man 2,5 bis 5,5 Äquivalente an Base bezogen auf 1 Äquivalent 4-Phenylbutanol einsetzt.

11. Verfahren nach einem der Ansprüche 1 bis 10, **dadurch gekennzeichnet, dass** man 0,01 bis 0,5 Äquivalente an Phasentransferkatalysator bezogen auf 1 Äquivalent 4-Phenylbutanol einsetzt.

12. Verfahren nach einem der Ansprüche 1 bis 11, **dadurch gekennzeichnet, dass** das Gemisch aus 1,6-Dibromhexan, einer Base, einem Phasentransferkatalysator und einem Verdünnungsmittels 1,5 bis 4,0 Volumenteile an Verdünnungsmittel bezogen auf ein Teil 1,6-Dibromhexan enthält.

13. Verfähren nach einem der Ansprüche 1 bis 12, **dadurch gekennzeichnet, dass** das Gemisch aus 4-Phenylbutanol und Verdünnungsmittel 1,5 bis 10 Volumeriteile an Verdünnungsmittel bezogen auf ein Teil 4-Phenylbutanol enthält.

14. Verfahren nach einem der Ansprüche 1 bis 13, **dadurch gekennzeichnet, dass** man nach Beendigung der Umsetzung Wasser zur Reaktionsmischung hinzufügt, die Phasen trennt, die organische Phase mit Wasser wäscht, unter reduziertem Druck einengt und den Rückstand unter Hochvakuum fraktioniert destilliert.

15. Verfahren zur Herstellung von Salmeterol, **dadurch gekennzeichnet, dass** das Edukt 4-(6-Bromhexyloxy)-butylbenzol nach einem der Ansprüche 1-14 hergestellt und in an sich bekannter Weise weiter verabeitet wird.

## Claims

1. Process for preparing 4-(6-bromohexyloxy)-butylbenzene by reacting 4-phenylbutanol with 1,6-dibromohexane in the presence of a base and a phase transfer catalyst, **characterised in that** 4-phenylbutanol in a diluent is metered into a mixture consisting of 1,6-dibromohexane, a base, a phase transfer catalyst and a diluent.

2. Process according to claim 1, **characterised in that** powdered potassium hydroxide is used as the base.

3. Process according to claim 1 or 2, **characterised in that** a tetraalkylammonium or tetraalkylphosphonium salt is used as the phase transfer catalyst.

4. Process according to claim 3, **characterised in that** a tetraalkylammonium hydrogen sulphate is used as the phase transfer catalyst.

5. Process according to one of claims 1 to 4, **characterised in that** an aromatic hydrocarbon or an optionally halogenated aliphatic hydrocarbon is used as diluent.

6. Process according to claim 5, **characterised in that** toluene or dichloromethane is used as diluent.

7. Process according to one of claims 1 to 6, **characterised in that** the reaction is carried out in a temperature range from -10°C to +30°C.

8. Process according to one of claims 1 to 7, **characterised in that** the 4-phenylbutanol is added within 10 to 240 minutes and the resulting reaction mixture is stirred for a further 30 to 180 minutes.

9. Process according to one of claims 1 to 8, **characterised in that** 0.75 to 2.5 equivalents of 1,6-dibromohexane are used, based on 1 equivalent of 4-phenylbutanol.

10. Process according to one of claims 1 to 9, **characterised in that** 2.5 to 5,5 equivalent of base are used, based on 1 equivalent of 4-phenylbutanol.

11. Process according to one of claims 1 to 10, **characterised in that** 0.01 to 0.5 equivalents of phase transfer catalyst are used, based on 1 equivalent of 4-phenylbutanol.

12. Process according to one of claims 1 to 11, **characterised in that** the mixture of 1,6-dibromohexane, a base, a phase transfer catalyst and a diluent contains 1.5 to 4.0 parts by volume of diluent, based on 1 part of 1,6-dibromohexane.

13. Process according to one of claims 1 to 12, **characterised in that** the mixture of 4-phenylbutanol and diluent contains 1.5 to 10 parts by volume of diluent, based on 1 part of 4-phenylbutanol.

14. Process according to one of claims 1 to 13, **characterised in that** after the reaction has ended water is added to the reaction mixture, the phases are separated, the organic phase is washed with water, concentrated under reduced pressure and the residue is fractionally distilled under high vacuum.

15. Process for preparing salmeterol, **characterised in that** the educt 4-(6-bromohexyloxy)-butylbenzene is prepared according to one of claims 1 to 14 and further processed in a manner known *per se*.

## Revendications

1. Procédé de préparation du 4-(6-bromohexyloxy)butylbenzène par réaction du 4-phénylbutanol avec le 1,6-dibromohexane en présence d'une base et d'un catalyseur de transfert de phase, **caractérisé en ce que** l'on ajoute du 4-phénylbutanol dans un diluant à un mélange consistant en 1,6-dibromohexane, une base, un catalyseur de transfert de phase et un diluant.

2. Procédé selon la revendication 1 **caractérisé en ce que** l'on utilise l'hydroxyde de potassium pulvérulent comme base.

3. procédé selon la revendication 1 ou 2 **caractérise en ce que** l'on utilise un sel de tétraalkylammonium ou de tétraalkylphosphonium comme catalyseur de transfert de phase.

4. Procédé selon la revendication 3 **caractérisé en ce que** l'on utilise un hydrogénosulfate de tétraalkylammonium comme catalyseur de transfert de phase.

5. Procédé selon l'une des revendications 1 à 4 **caractérisé en ce que** l'on utilise comme diluant un hydrocarbure aromatique ou un hydrocarbure aliphatique éventuellement halogéné.

6. Procédé selon la revendication 5 **caractérisé en ce que** l'on utilise le toluène ou le dichlorométhane comme diluant.

7. Procédé selon l'une des revendications 1 à 6 **caractérisé en ce que** l'on conduit la réaction dans un domaine de température de -10°C à +30°C.

8. Procédé selon l'une des revendications 1 à 7 **caractérisé en ce que** l'on ajoute le 4-phénylbutanol en 10 à 240 minutes et on agite le mélange réactionnel obtenu pendant encore 30 à 180 minutes supplémentaires.

9. Procédé selon l'une des revendications 1 à 8 **caractérisé en ce que** l'on utilise 0,75 à 2,5 équivalents de 1,6-dibromohexane par équivalent de 4-phénylbutanol.

10. Procédé selon l'une des revendications 1 à 9 **caractérisé en ce que** l'on utilise 2,5 à 5,5 équivalents de base par équivalent de 4-phénylbutanol.

11. Procédé selon l'une des revendications 1 à 10 **caractérisé en ce que** l'on utilise 0,01 à 0,5 équivalent de catalyseur de transfert de phase par équivalent de 4-phénylbutanol.

12. Procédé selon l'une des revendications 1 à 11 **caractérisé en ce que** le mélange de 1,6-dibromohexane, d'une base, d'un catalyseur de transfert de phase et d'un diluant contient 1,5 à 4,0 parties en volume de diluant par partie de 1,6-dibromohexane.

13. Procédé selon l'une des revendications 1 à 12 **caractérisé en ce que** le mélange de 4-phénylbutanol et de diluant contient 1,5 à 10 parties en volume de diluant par partie de 4-phénylbutanol.

14. Procédé selon l'une des revendications 1 à 13 **caractérisé en ce que**, après la fin de la réaction, on ajoute de l'eau au mélange réactionnel, on sépare les phases, on lave à l'eau la phase organique, on concentre sous pression réduite et on soumet le résidu à une distillation fractionnée sous haut vide.

15. Procédé de préparation du salmétérol **caractérisé en ce que** le produit de départ 4-(6-bromohexyloxy)-butylbenzène est préparé selon l'une des revendications 1-14 et traité encore de manière connue en soi.
